# EUROPEAN PATENT APPLICATION

(11) **EP 3 492 113 A1**
(43) Date of publication of application: **05.06.2019**
(21) Application number: 18209764.2
(22) Date of filing: 03.12.2018
(51) Int. Cl.: A61L 2/20, A61B 1/00, A61L 2/26, B65D 75/00

(54) **STERILIZATION PACKAGE**

(30) Priority: 04.12.2017 US 201715830331
(71) Applicant: Ethicon, Inc., Somerville, NJ 08876 (US)
(72) Inventor: FRYER, Ben, Irvine, CA California 92618 (US)
(74) Representative: Small, Gary James

(57) **Abstract**

A sterilization package for an endoscope that is described herein. The sterilization package may be used for endoscopes over six feet long. The sterilization package may include a first leg, a second leg connected to the first leg, an opening, and a flap disposed proximate the opening and configured to cover the opening. The package may be 95% by weight of a vapor permeable material. The package, with the endoscope disposed therein, may be coiled and placed into a vacuum chamber of a sterilization system.

## Description

### FIELD

The subject matter disclosed herein relates to sterilizing endoscopes.

### BACKGROUND

Endoscopes are reusable medical devices. An endoscope should be reprocessed, i.e., decontaminated, between medical procedures in which it is used to avoid causing infection or illness in a subject. Endoscopes are difficult to decontaminate as has been documented in various news stories. *See*, *e*.*g*., Chad Terhune, "Superbug outbreak: UCLA will test new scope-cleaning machine," LA Times, July 22, 2015, http://www.latimes.com/business/la-fi-ucla-superbug-scope-testing-20150722-story.html (last visited October 30, 2017). Typically, endoscope reprocessing is performed by a disinfection procedure that includes at least the following steps: removing foreign material from the endoscope, cleaning the endoscope, and disinfecting the endoscope by submerging it in a disinfectant capable of substantially killing microorganisms thereon, e.g., infection causing bacteria. One exemplary disinfectant is CIDEX® OPA Solution, manufactured and distributed by Applicant, Advanced Sterilization Products, Division of Ethicon US, LLC, a Johnson & Johnson company ("ASP").

Many healthcare facilities including hospitals decontaminate medical devices by sterilization procedures. Various sterilization techniques may be employed, such as steam, hydrogen peroxide, and vapor phase sterilization, either with or without a gas plasma and ethylene oxide (EtO). Each of these methods depends to a certain extent on the diffusion rates of the sterilization fluids, typically gases, upon the medical devices to be sterilized. Certain sterilization techniques are conducted at pressures other than ambient pressure or atmospheric pressure. For example the STERRAD® System, STERRAD® NX System or STERRAD® 100NX System of ASP, are examples of sterilization systems that vaporize hydrogen peroxide and operate at low pressures, e.g., less than 200 millitorr.

Sterilization procedures are generally regarded as being more effective than disinfection procedures at killing microorganisms. However, sterilizing endoscopes effectively has various shortcomings. These shortcomings have to date minimized the likelihood that a healthcare facility, such as hospital, will opt to sterilize endoscopes instead of disinfect endoscopes.

One such shortcoming arises when an endoscope must be coiled to fit into a sterilization chamber. Endoscopes used on human subjects may be over approximately 10 to 12 feet long, whereas a vacuum chamber in a sterilizer-into which the endoscope must fit-may be much shorter. For example, a vacuum chamber of the STERRAD® 100 NX System has dimensions of approximately 2 feet x 1.5 feet x 0.5 feet and the STERRAD® NX System has dimensions of approximately 2 feet x 1 ft x 0.5 feet. Accordingly, an endoscope that is about 10 feet long likely would need to be configured into a coil having about five convolutions to fit into the vacuum chambers. Those portions of the endoscope that touch other portions of the endoscope or other materials within the sterilization chamber (e.g., a sterilization tray in which it is placed, a wall of the chamber, impermeable portions of a sterilization pouch) may be referred to as "mated surfaces." Mated surfaces are difficult to sterilize relative to exposed, i.e., non-mated surfaces because an adequate amount of sterilant to kill the bacteria on the mated surfaces may not reach those mated surfaces within a reasonable amount of time. Endoscopes are typically fabricated from polyurethane, which is vapor absorptive, such that it may absorb a gaseous sterilant, including hydrogen peroxide, before the sterilant can penetrate through the endoscope to the mated surfaces. Thus, the mated surfaces may not be sufficiently exposed to the sterilant to effectively kill the bacteria.

### SUMMARY

A sterilization package is described herein. The sterilization package is suited to disposing an endoscope therein. The sterilization package may include a first leg, a second leg connected to the first leg, an opening, and a flap disposed proximate the opening and configured to cover the opening. The package, and particularly the first leg, the second leg, and the flap may be fabricated from a vapor permeable material. The package may be at least 95% by weight of the material. The first leg may have a first length, the second leg may have a second length, and the first length plus the second length may equal at least approximately six feet. The vapor-permeable material may include a non-woven fabric, including melt flow, spun-bonded or flashspun materials, e.g., flashspun high-density polyethylene fibers and/or non-woven polypropylene fabric.

An adhesive may be disposed upon the flap. The adhesive may be adapted to seal the flap about the opening. The opening may be a single opening disposed in the first leg proximate the second leg. The package may lack any other openings.

Also disclosed herein is a method for sterilizing an endoscope in a sterilization package, such as the sterilization package described above. The method may be used to sterilize endoscopes that are at least six feet long. The endoscope may be placed into the package. The flap may be sealed over the opening of the package. The package, with the endoscope disposed therein, may be placed into a vacuum chamber of a sterilization system. The package, with the endoscope disposed therein, may be coiled before, during, or after placing the package in the vacuum chamber. The coiled package and endoscope may be checked to confirm that no portion of the endoscope contacts another portion of the endoscope. The coiled package and endoscope may be placed in the vacuum chamber without placing a sterilization tray in the vacuum chamber.

### BRIEF DESCRIPTION OF THE DRAWINGS

While the specification concludes with claims which particularly point out and distinctly claim the subject matter described herein, it is believed the subject matter will be better understood from the following description of certain examples taken in conjunction with the accompanying drawings, in which like reference numerals identify the same elements and in which:
Figure 1A depicts an endoscope;
Figure 1B depicts the endoscope of Figure 1A in a coiled configuration;
Figure 2 depicts a sterilization package; and
Figure 3 depicts the endoscope of Figure 1A, coiled inside the sterilization package of Figure 2.

### DETAILED DESCRIPTION

The following description sets forth certain illustrative examples of the claimed subject matter. Other examples, features, aspects, embodiments, and advantages of the technology should become apparent to those skilled in the art from the following description. Accordingly, the drawings and descriptions should be regarded as illustrative in nature.

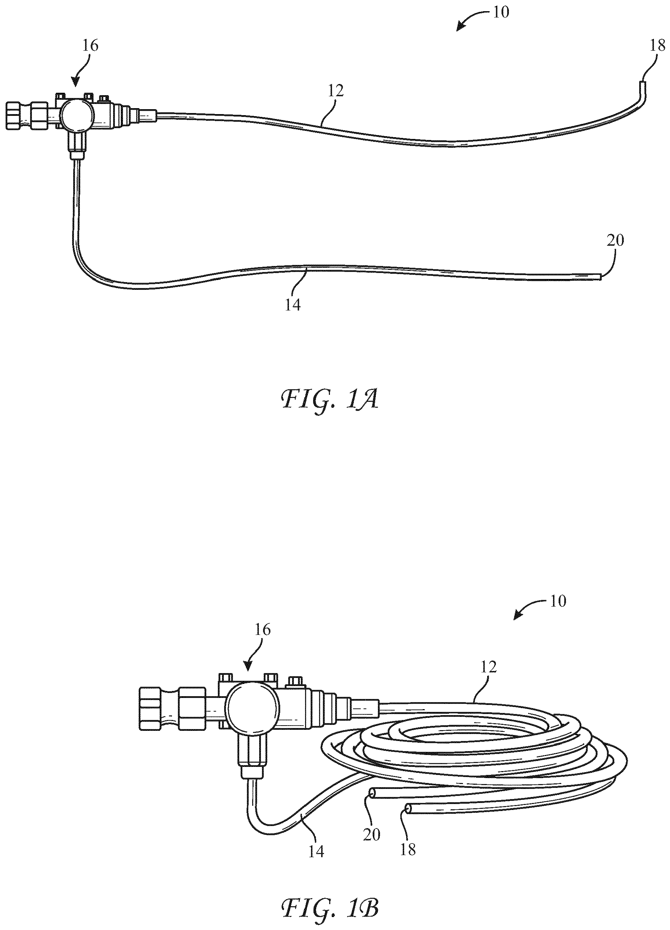

Figure 1A shows an exemplary endoscope 10. An endoscope typically comprises an insertion tube or first tube 12 and a second tube 14, both connected to a body 16. Although endoscopes come in many lengths, as measured from first end 18 of first tube 12 to second end 20 of second tube 14, the present disclosure focuses on endoscopes that are at least six feet long, because endoscopes that are at least six feet long likely must be coiled to fit into a sterilization chamber, thus creating the mated-surfaces problem described above. Figure 1B shows endoscope 10 in a coiled configuration. Significant portions of endoscope 10 are covered by other portions of endoscope 10, creating mated surfaces.

Uncoiled, endoscope 10 is difficult to fit inside the vacuum chamber of a sterilization system that a hospital is likely to own and use, e.g., the STERRAD® 100 NX System or the STERRAD® NX System. Coiled, the endoscope may fit therein, but a sterilization cycle may fail to sterilize the endoscope because the sterilant may not reach the mated surfaces or penetrate through the endoscope material in the vicinity of the mated surfaces.

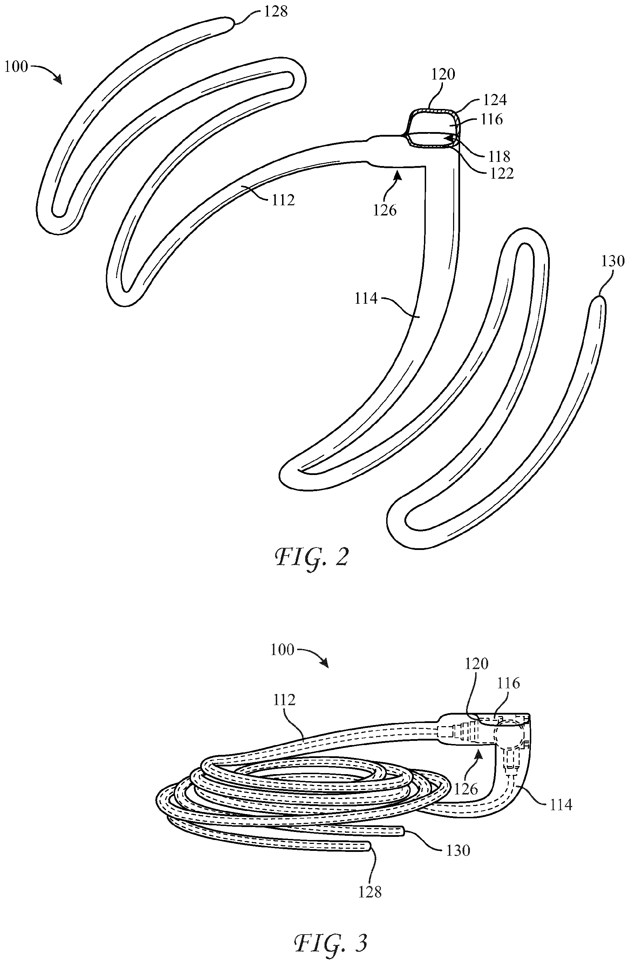

Figure 2 shows a sterilization package 100 that helps prevent an endoscope from contacting itself when coiled. Package 100 may be used to increase the likelihood that an endoscope may be sterilized. Sterilization pack 100 includes a first leg 112, a second leg 114, a flap 116, and an opening 118 disposed thorough first leg 112 and proximate flap 116. Flap 116 may be brought into contact with first leg 112, such that edge 120 of flap 116 may contact edge 122 of opening 118. An adhesive or sealant 124 may be disposed on edge 120 and/or edge 122 such that flap 116 seals opening 118 upon contact between edge 120 and edge 122. Sterilization package 100 has a shape similar to endoscope 10 such that endoscope may be inserted therein, likely via opening 118, and such that first leg 112 conforms closely to first tube 12 and second leg 114 conforms closely to second leg 14. In some embodiments, a portion 126 of first leg 112 may be larger than other portions of first leg 112 to facilitate placement of body 16 therein. Opening 118 may be disposed through larger portion 126 and flap 116 may be disposed proximate thereto to further facilitate inserting endoscope 10 into package 100 and to facilitate sealing opening 118 with flap 116.

First leg and second leg may be sealed or unsealed at their respective ends 128 and 130. The ends may be sealed during manufacturing, or sealed by healthcare personnel before or after endoscope 10 is inserted into package 100. Healthcare personnel may find it easier to place endoscope 10 into package 100 if ends 128 and 130 are not sealed by the manufacturer. Irrespective of whether ends 128 and 130 are unsealed or sealed by the manufacturer, opening 118 should always be sealed using flap 116 by healthcare personnel after endoscope 10 is placed fully into package 100. When ends 128 and 130 are sealed by the manufacturer, package 100 has opening 118 is the only opening in package 100. Package 100 should comprise a vapor permeable material. Suitable materials include, e.g., flashspun high-density polyethylene fibers, including DuPont™ Tyvek®, and non-woven polypropylene fabric, including KIMBERLY-CLARK* KIMGUARD* Sterilization Wrap. Ideally, Package 100 should minimize use of vapor-impermeable materials, including the impermeable polymer materials that are often included in, e.g., breathable sterilization tear pouches, e.g., Qosina P/N 91200. Such materials include, e.g., polyesters and low-density polyethylene films.

By maximizing the amount of vapor permeable material and minimizing the amount of vapor impermeable material of which package 100 is comprised, package 100 enables sterilant to reach an endoscope's surfaces by helping to remove mated surfaces. In some embodiments, package 100 is comprised entirely of vapor permeable material or materials. In some embodiments, package 100 is comprised of at least 95% vapor permeable material by weight and/or volume. Vapor impermeable materials may be included in small quantities to assist with sealing package 100 or to view endoscope 10 within package 100 when sealed. However, vapor impermeable materials should be minimized, particularly near to polyurethane portions of the endoscope, to avoid introduction of mated surfaces between the endoscope and impermeable portions of the package. As shown in Figure 3, endoscope 10 may be disposed within package 100, flap 116 may seal opening 118, and the combination of endoscope 10 and package 100 may be placed into a coiled configuration. In the coiled configuration, endoscope 10 may fit within a vacuum chamber of a sterilization system. So coiled, two layers of package 100 are disposed any portions of endoscope 10 that would otherwise contact to form a mated surface. Thus, no portion of endoscope 10 contacts any other portions the endoscope, few or no portions of endoscope 10 touch a vapor impermeable portion of sterilization package 10, and no portion of endoscope 10 will touch a sterilization tray or a wall of a vacuum chamber. Two layers of package 100 provides sufficient separation about endoscope 10 to allow a sterilant to reach those surfaces that would otherwise be mated with vapor impermeable or vapor absorptive surfaces.

The inventor has discovered an additional benefit of using package 100, i.e., that use of package 100 results in improved removal of residual moisture upon or within the endoscope, that might otherwise inhibit sterilization of the endoscope. Typically, instruments to be sterilized are placed into a sterilization tray, which is then wrapped with a sterilization wrap. The tray and the wrap are of substantial mass that they inhibit conduction of energy from walls or shelving of the vacuum chamber to the endoscope. Accordingly, this energy cannot assist vaporization of residual moisture. Package 100 removes the need to use a wrapped sterilization tray, it is lightweight, and a poor insulator. For those reasons, endoscope 10 may be sterilized in package 100 but not in a sterilization tray such that endoscope 10 within package 100 may be placed into a vacuum chamber of a sterilization system without a sterilization tray. This may enable greater conduction of energy to the endoscope and improved removal of residual moisture.

In light of the foregoing, package 100 may be used according to the following exemplary steps. First, package 100 and endoscope 10 are provided. Second, first tube 12 is positioned inserted through opening 118 and into first leg 112. Third, second tube 14 is inserted through opening 118 and into second leg 114. Fourth, body 16 is disposed through opening 118 and into portion 126. Fifth, opening 118 is sealed by adhering flap 116 over it. In those instances where ends 128 and 130 of first leg and second leg are unsealed, healthcare personnel may seal them. Sixth, endoscope 10 along with package 100 are together coiled. Seventh, endoscope 10 along with package 100 are placed, in a coiled configuration, into a vacuum chamber of a sterilization system. This step may be performed with endoscope 10 in a sterilization tray. Alternatively, this step may be performed without a sterilization tray. That is, endoscope 10 in package 100 may be placed directly into the vacuum chamber without using a sterilization tray.

It should be understood that any of the examples and/or embodiments described herein may include various other features in addition to or in lieu of those described above. The teachings, expressions, embodiments, examples, etc. described herein should not be viewed in isolation relative to each other. Various suitable ways in which the teachings herein may be combined should be readily apparent to those of ordinary skill in the art in view of the teachings herein.

Having shown and described exemplary embodiments of the subject matter contained herein, further adaptations of the methods and systems described herein may be accomplished by appropriate modifications without departing from the scope of the claims. Some such modifications should be apparent to those skilled in the art. For instance, the examples, embodiments, geometries, materials, dimensions, ratios, steps, and the like discussed above are illustrative. Accordingly, the claims should not be limited to the specific details of structure and operation set forth in the written description and drawings.

## Claims

1. A sterilization package, comprising:
a first leg;
a second leg connected to the first leg;
an opening; and
a flap disposed proximate the opening and configured to cover the opening,
wherein the first leg, the second leg, and the flap are fabricated from a vapor-permeable material such that the package is at least 95% by weight of the material, and
wherein the first leg has a first length, the second leg has a second length, and the first length plus the second length equals a least approximately six feet.

2. A method for sterilizing an endoscope, comprising:
providing a package, the package comprising,
a first leg;
a second leg connected to the first leg;
an opening; and
a flap disposed proximate the opening and configured to cover the opening;
wherein the first leg, the second leg, and the flap are fabricated from a vapor-permeable material such that the package is at least 95% by weight of the material, and
wherein the first leg has a first length, the second leg has a second length,
and the first length plus the second length equals a least approximately six feet, placing an endoscope that is at least approximately six feet long within the package;
sealing the package;
placing the endoscope and package into a vacuum chamber of a sterilization system; and
passing a sterilant through the package.

3. The method of claim 2, wherein the step of placing the endoscope and package in the vacuum chamber includes coiling the endoscope and package.

4. The method of claim 3, further comprising confirming that no portion of the endoscope contacts another portion of the endoscope.

5. The package of claim 1 or the method of claim 4, wherein the material comprises a non-woven fabric

6. The package or the method of claim 5, wherein the non-woven fabric includes flashspun high-density polyethylene fibers.

7. The package or the method of claim 5, wherein the non-woven fabric is a non-woven polypropylene fabric.

8. The package of claim 5, further comprising an adhesive disposed upon the flap.

9. The package of claim 8, wherein the adhesive is adapted to seal the flap about the opening.

10. The method of claim 5, wherein the package further includes an adhesive disposed upon the flap.

11. The method of claim 10, wherein the adhesive is adapted to seal the flap about the opening and the step of sealing the package includes covering the opening with the flap.

12. The package of claim 8 or the method of claim 10, wherein the opening is a single opening disposed in the first leg proximate the second leg.

13. The package or the method of claim 12, wherein the package lacks any other openings.

14. The package of claim 12, further comprising an endoscope disposed within the package.

15. The method of claim 2, wherein the step of placing the endoscope and package into a vacuum chamber of a sterilization system is performed without placing a sterilization tray into the vacuum chamber.
